(19) **Europäisches Patentamt / European Patent Office / Office européen des brevets**

(11) **EP 4 181 891 B1**

(12) **EUROPEAN PATENT SPECIFICATION**

(45) Date of publication and mention
of the grant of the patent:
**17.12.2025   Bulletin 2025/51**

(21) Application number: **21746671.3**

(22) Date of filing: **15.07.2021**

(51) International Patent Classification (IPC):
*A61K 31/05* (2006.01)      *A61K 31/19* (2006.01)
*A61K 31/195* (2006.01)     *A61K 31/197* (2006.01)
*A61K 31/4015* (2006.01)    *A61K 31/4192* (2006.01)
*A61K 31/53* (2006.01)      *A61K 31/551* (2006.01)
*A61K 31/7048* (2006.01)    *A61P 25/08* (2006.01)
*A61K 45/06* (2006.01)      *A61K 36/185* (2006.01)

(52) Cooperative Patent Classification (CPC):
(C-Sets available)
**A61P 25/08; A61K 31/05; A61K 31/19;
A61K 31/195; A61K 31/197; A61K 31/4015;
A61K 31/4192; A61K 31/53; A61K 31/551;
A61K 31/7048; A61K 36/185; A61K 45/06**   (Cont.)

(86) International application number:
**PCT/EP2021/069794**

(87) International publication number:
**WO 2022/017911 (27.01.2022 Gazette 2022/04)**

(54) **USE OF CANNABIDIOL IN THE TREATMENT OF ABSENCE SEIZURES ASSOCIATED WITH RARE EPILEPSY SYNDROMES RELATED TO BRAIN INJURY**

VERWENDUNG VON CANNABIDIOL BEI DER BEHANDLUNG VON ABSENCE-ANFÄLLEN BEI SELTENEN EPILEPSIESYNDROMEN, DIE MIT GEHIRNVERLETZUNG VERBUNDEN SIND

UTILISATION DU CANNABIDIOL DANS LE TRAITEMENT DES CRISES D'ABSENCE ASSOCIÉES AUX SYNDROMES D'ÉPILEPSIE RARES LIÉS AUX LÉSIONS CÉRÉBRALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **20.07.2020   GB 202011123**

(43) Date of publication of application:
**24.05.2023   Bulletin 2023/21**

(73) Proprietor: **Jazz Pharmaceuticals Research UK Limited
Sittingbourne, Kent ME9 8AG (GB)**

(72) Inventors:
• **CHECKETTS, Daniel, Adam
Sittingbourne
Kent
ME9 8AG (GB)**

• **CRAIG, Kevin, James
Sittingbourne
Kent
ME9 8AG (GB)**

(74) Representative: **HGF
HGF Limited
4th Floor, 1 City Square
Leeds LS1 2AL (GB)**

(56) References cited:
**WO-A1-2019/207319**

EP 4 181 891 B1

- ALVAREZ FRANCISCO J ET AL: "Neuroprotective Effects of the Nonpsychoactive Cannabinoid Cannabidiol in Hypoxic-Ischemic Newborn Piglets", PEDIATRIC RESEARCH, LIPPINCOTT WILLIAMS & WILKINS, NEW YORK, US, vol. 64, no. 6, December 2008 (2008-12-01), pages 653 - 658, XP008163929, ISSN: 0031-3998, DOI: 10.1203/PDR.0B013E318186E5DD
- SULAK DUSTIN ET AL: "The current status of artisanal cannabis for the treatment of epilepsy in the United States", EPILEPSY AND BEHAVIOR, ACADEMIC PRESS, SAN DIEGO, CA, US, vol. 70, 21 February 2017 (2017-02-21), pages 328 - 333, XP085033470, ISSN: 1525-5050, DOI: 10.1016/J.YEBEH.2016.12.032
- TZADOK MICHAL ET AL: "CBD-enriched medical cannabis for intractable pediatric epilepsy The current Israeli experience", SEIZURE, BAILLIERE TINDALL, LONDON, GB, vol. 35, 6 January 2016 (2016-01-06), pages 41 - 44, XP029421038, ISSN: 1059-1311, DOI: 10.1016/J.SEIZURE.2016.01.004
- DIZON MARIA L. V. ET AL: "Practice variation in anti-epileptic drug use for neonatal hypoxic-ischemic encephalopathy among regional NICUs", BMC PEDIATRICS, vol. 19, no. 1, 27 February 2019 (2019-02-27), pages 67, XP055847375, DOI: 10.1186/s12887-019-1441-7

(52) Cooperative Patent Classification (CPC): (Cont.)

C-Sets
A61K 31/05, A61K 2300/00;
A61K 31/19, A61K 2300/00;
A61K 31/195, A61K 2300/00;
A61K 31/197, A61K 2300/00;
A61K 31/4015, A61K 2300/00;
A61K 31/4192, A61K 2300/00;
A61K 31/53, A61K 2300/00;
A61K 31/551, A61K 2300/00;
A61K 31/7048, A61K 2300/00

## Description

### FIELD OF THE INVENTION

The present invention relates to the use of cannabidiol (CBD) for the treatment of absence seizures associated with hypoxic ischaemic encephalopathy (HIE) or anoxia at birth. Other types of seizures include tonic, tonic-clonic, atonic, myoclonic, absence, focal seizures with impairment and spasms. Preferably the dose of CBD is between 5 mg/kg/day to 50 mg/kg/day.

In a further embodiment the CBD used is in the form of a highly purified extract of cannabis such that the CBD is present at greater than 95% of the total extract (w/w) and the cannabinoid tetrahydrocannabinol (THC) has been substantially removed, to a level of not more than 0.15% (w/w).

Preferably the CBD used is in the form of a botanically derived purified CBD which comprises greater than or equal to 98% (w/w) CBD and less than or equal to 2% (w/w) of other cannabinoids. More preferably the other cannabinoids present are THC at a concentration of less than or equal to 0.1% (w/w); CBD-C1 at a concentration of less than or equal to 0.15% (w/w); CBDV at a concentration of less than or equal to 0.8% (w/w); and CBD-C4 at a concentration of less than or equal to 0.4% (w/w). The botanically derived purified CBD preferably also comprises a mixture of both trans-THC and cis-THC. Alternatively, a synthetically produced CBD is used.

Most preferably the other cannabinoids present are THC at a concentration of about 0.01% to about 0.1% (w/w); CBD-C1 at a concentration of about 0.1% to about 0.15% (w/w); CBDV at a concentration of about 0.2% to about 0.8% (w/w); and CBD-C4 at a concentration of about 0.3% to about 0.4% (w/w). Most preferably still the THC is present at a concentration of about 0.02% to about 0.05% (w/w).

Where the CBD is given concomitantly with one or more other anti-epileptic drugs (AED), the CBD may be formulated for administration separately, sequentially or simultaneously with one or more AED or the combination may be provided in a single dosage form.

### BACKGROUND TO THE INVENTION

Epilepsy occurs in approximately 1% of the population worldwide, (Thurman *et al.,* 2011) of which 70% are able to adequately control their symptoms with the available existing anti-epileptic drugs (AED). However, 30% of this patient group, (Eadie *et al.,* 2012), are unable to obtain seizure freedom from the AED that are available and as such are termed as suffering from intractable or "treatment-resistant epilepsy" (TRE).

Intractable or treatment-resistant epilepsy was defined in 2009 by the International League Against Epilepsy (ILAE) as *"failure of adequate trials of two tolerated and appropriately chosen and used AED schedules (whether as monotherapies or in combination) to achieve sustained seizure freedom"* (Kwan *et al.,* 2009).

Individuals who develop epilepsy during the first few years of life are often difficult to treat and as such are often termed treatment resistant. Children who undergo frequent seizures in childhood are often left with neurological damage which can cause cognitive, behavioral and motor delays.

Childhood epilepsy is a relatively common neurological disorder in children and young adults with a prevalence of approximately 700 per 100,000. This is twice the number of epileptic adults per population.

When a child or young adult presents with a seizure, investigations are normally undertaken in order to investigate the cause. Childhood epilepsy can be caused by many different syndromes and genetic mutations and as such diagnosis for these children may take some time.

The main symptom of epilepsy is repeated seizures. In order to determine the type of epilepsy or the epileptic syndrome that a patient is suffering from an investigation into the type of seizures that the patient is experiencing is undertaken. Clinical observations and electroencephalography (EEG) tests are conducted and the type(s) of seizures are classified according to the ILEA classification.

Generalized seizures, where the seizure arises within and rapidly engages bilaterally distributed networks, can be split into six subtypes: tonic-clonic (grand mal) seizures; absence (petit mal) seizures; clonic seizures; tonic seizures; atonic seizures and myoclonic seizures.

Focal (partial) seizures where the seizure originates within networks limited to only one hemisphere, are also split into sub-categories. Here the seizure is characterized according to one or more features of the seizure, including aura, motor, autonomic and awareness / responsiveness. Where a seizure begins as a localized seizure and rapidly evolves to be distributed within bilateral networks this seizure is known as a bilateral convulsive seizure, which is the proposed terminology to replace secondary generalized seizures (generalized seizures that have evolved from focal seizures and are no longer remain localized).

Focal seizures where the subject's awareness / responsiveness is altered are referred to as focal seizures with impairment and focal seizures where the awareness or responsiveness of the subject is not impaired are referred to as focal seizures without impairment.

Hypoxic ischemic encephalopathy (HIE) or anoxia at birth is a type of brain dysfunction that occurs when the brain doesn't receive enough oxygen or blood flow for a period of time. Hypoxic means not enough oxygen; ischemic means not enough blood flow; and encephalopathy means brain disorder.

HIE may develop during pregnancy, labour and delivery or in the postnatal period.

As the brain injury occurs during the neonatal period there is a range of different symptoms and co-morbid conditions that are likely to occur as the child gets older. Some children will experience no health issues or only mild or moderate effects, while others have much more severe and permanent disability, such as developmental delay; cerebral palsy (motor impairment); epileptic seizures; or cognitive impairment.

Symptoms of HIE usually depend on the severity and extent of the brain injury, as well as the areas of the brain that were affected. Babies born with HIE may be floppy and unreactive to sights or sounds. Alternatively, some babies with HIE are very tense and react more to stimulation than a healthy newborn. In addition, they are likely to suffer from abnormal movements or seizures.

Treatment is usually started immediately after birth by cooling the baby for three days on a cooling blanket. Treatment for the co-morbid conditions such as motor impairment or epilepsy is started as the child gets older.

If the blood or oxygen supply to the brain has been interrupted, the rest of the body may have also been "starved" of oxygen. This may cause damage to other organs, including the heart, liver, kidneys and bowels. These organs usually return to normal function. However, if the brain has sustained an injury, it may not recover fully. The length of time the brain was without oxygen usually determines the severity of the damage.

Cannabidiol (CBD), a non-psychoactive derivative from the cannabis plant, has demonstrated anti-convulsant properties in several anecdotal reports, pre-clinical and clinical studies both in animal models and humans. Three randomized control trials showed efficacy of the purified pharmaceutical formulation of CBD in patients with Dravet and Lennox-Gastaut syndrome.

Based on these three trials, a botanically derived purified CBD preparation was approved by FDA in June 2018 for the treatment of seizures associated with Dravet and Lennox-Gastaut syndromes.

Preclinical studies have shown that CBD may be neuroprotective[1] and this has led to a clinical trial whereby newborn babies with HIE are administered CBD in addition to hypothermia at birth[2].

However, these studies are testing the use of CBD in preventing HIE rather than its use in the treatment of co-morbid symptoms which develop after the brain injury such as seizures.

In 2008 Morales *et al.*[3] investigated the use of 100% CBD to treat seizures in patients with Lennox Gastaut syndrome half of whom also presented with HIE. It was reported that a reduction in seizures was obtained for the LGS patients.

Zollner *et al.* (2019)[4] describes a case study of an adult patient who presented with treatment-resistant post-hypoxic myoclonus secondary to cerebra anoxia, who was subsequently diagnosed with Lance-Adams syndrome. It is disclosed that CBD was used in combination with other antiepileptics, with no reference to the specific composition of CBD used.

Alvarez *et al.*(2008)[5] discloses a study whereby new-born piglets with HIE were administered CBD due to its possible neuroprotective effect. A reduction in seizures was reported, but half of the CBD-treated piglets still maintained significant seizures.

GB 2551986 describes the use of an aqueous parenteral cannabinoid containing formulation which may be used to treat newborn hypoxic-ischemic encephalopathy (NHIE), status epilepticus, or stroke due to their neuroprotective and anti-convulsant effect.

Additionally, GB 2504263 relates to the combination of therapeutic hypothermia with CBD which has been shown to be synergistic in neuroprotection following HI injury in an animal model of NHIE.

GB 2574321 describes a highly purified CBD composition, with the THC being present as a mixture of trans and cis-THC, for use as a medicament in a broad spectrum of neurodevelopmental diseases and conditions.

Sulak D et al. (Epilepsy and Behavior, 2017, 70:328-333) reports on artisanal cannabis preparations for use in the treatment of medically refractory epilepsy.

The applicant has found by way of an open label, expanded-access program that treatment with CBD resulted in a significant reduction in specific seizure types including tonic, tonic-clonic, atonic, myoclonic, absence, focal seizures with impairment and spasms in patients diagnosed with hypoxic ischaemic encephalopathy (HIE) or anoxia at birth.

## BRIEF SUMMARY OF THE DISCLOSURE

In accordance with a first aspect of the present invention there is provided a cannabidiol (CBD) preparation for use in the treatment of absence seizures associated with hypoxic ischaemic encephalopathy (HIE) or anoxia at birth.

Other seizures associated with hypoxic ischaemic encephalopathy (HIE) or anoxia at birth are tonic, tonic-clonic, atonic, myoclonic, focal seizures with impairment and spasms.

In a further embodiment, the CBD preparation comprises greater than 95% (w/w) CBD and not more than 0.15% (w/w) tetrahydrocannabinol (THC).

Preferably the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC.

Preferably the CBD preparation is used in combination with one or more concomitant anti-epileptic drugs (AED).

Preferably the one or more AED is selected from the group consisting of: valproic acid, levetiracetam, clobazam, vigabatrin, rufinamide, topiramate, lamotrigine and gabapentin.

In one embodiment the CBD is present is isolated from cannabis plant material. Preferably at least a portion of at least one of the cannabinoids present in the CBD preparation is isolated from cannabis plant material.

In a further embodiment the CBD is present as a synthetic preparation. Preferably at least a portion of at least one of the cannabinoids present in the CBD preparation is prepared synthetically.

Preferably the dose of CBD is greater than 5 mg/kg/day. More preferably the dose of CBD is 20 mg/kg/day. More preferably the dose of CBD is 25 mg/kg/day. More preferably the dose of CBD is 50 mg/kg/day.

## DEFINITIONS

Definitions of some of the terms used to describe the invention are detailed below:

Over 100 different cannabinoids have been identified, see for example, Handbook of Cannabis, Roger Pertwee, Chapter 1, pages 3 to 15. These cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoids" are defined as cannabinoids that have been extracted from the cannabis plant and purified to the extent that other cannabinoids and non-cannabinoid components that are co-extracted with the cannabinoids have been removed, such that the highly purified cannabinoid is greater than or equal to 95% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED.

"Tonic seizures" can be generalised onset, affecting both sides of the brain, or they can be focal onset, starting in just one side of the brain. If a tonic seizure starts in both sides of the brain, all muscles tighten and the subject's body goes stiff. If standing, they may fall to the floor, their neck may extend, eyes open wide and roll upwards, whilst their arms may raise upwards and legs stretch or contract. If a tonic seizure starts in one side of the brain muscles tighten in just one area of the body. Tonic seizures usually last less than one minute.

"Tonic-clonic seizures" consist of two phases: the tonic phase and the clonic phase. In the tonic phase the body becomes entire rigid, and in the clonic phase there is uncontrolled jerking. Tonic-clonic seizures may or may not be preceded by an aura, and are often followed by headache, confusion, and sleep. They may last mere seconds or continue for several minutes. These seizures are also known as a grand mal seizure.

"Atonic seizures" occur when a person suddenly loses muscle tone so their head or body may go limp. They are also known as drop attacks. In some children, only their head drops suddenly. They can begin in one area or side of the brain (focal onset) or both sides of the brain (generalized onset).

"Myoclonic seizures" are characterised by a 'muscle jerk'. Myoclonic seizures are brief but can happen in clusters (many happening close together in time) and often happen shortly after waking. In myoclonic seizures the person is conscious, but they are classified as generalised seizures.

"Absence seizures" also may be called "petit mal" seizures. These types of seizure cause a loss of awareness for a short time. They mainly affect children although can happen at any age. During an absence seizure, a person may: stare blankly into space; look like they're "daydreaming"; flutter their eyes; make slight jerking movements of their body or limbs. The seizures usually only last up to 15 seconds and may occur several times a day.

"Focal Seizures" are defined as seizures which originate within networks limited to only one hemisphere. What happens during the seizure depends on where in the brain the seizure happens and what that part of the brain normally does.

"Focal seizures without impairment" are seizures which originate within networks limited to only one hemisphere where the awareness or responsiveness of the subject is not impaired.

"Focal seizure with impairment" usually start in a small area of the temporal lobe or frontal lobe of the brain and involve other areas of the brain within the same hemisphere that affect alertness and awareness. Most subjects experience automatisms during a focal seizure with impaired consciousness.

"Focal seizure with secondary generalisation" start in a limited area on one side of the brain and spread to involve both sides. This is different from a generalized onset seizure, which starts on both sides of the brain.

"Epileptic spasm", "spasms", "infantile spasm", "juvenile spasm" or "West syndrome" is defined as sudden flexion, extension or mixed flexion-extension of proximal and truncal muscles, lasting 1-2 seconds. Spasms typically occur in a series, usually on wakening. Subtle forms may occur with only chin movement, grimacing, or head nodding. Spasms may be bilaterally symmetric, asymmetric, or unilateral, depending on whether they are generalised onset or focal onset.

## DETAILED DESCRIPTION

### PREPARATION OF HIGHLY PURIFIED CBD EXTRACT

The following describes the production of the highly-purified (>95% w/w) cannabidiol extract which has a known and constant composition.

In summary the drug substance used is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than or equal to 95% CBD. Although the CBD is highly purified because it is produced from a cannabis plant rather than synthetically there is a small number of other cannabinoids which are co-produced and co-extracted with the CBD. Details of these cannabinoids and the quantities in which they are present in the medication are as described in Table A below.

Table A: Composition of highly purified CBD extract

| Cannabinoid | Concentration |
|---|---|
| CBD | > 95% w/w |
| CBDA | NMT 0.15% w/w |
| CBDV | NMT 1.0% w/w |
| $\Delta^9$ **THC** | NMT 0.15% w/w |
| CBD-C4 | NMT 0.5% w/w |
| > - greater than<br>NMT - not more than | |

### PREPARATION OF BOTANICALLY DERIVED PURIFIED CBD

The following describes the production of the botanically derived purified CBD which comprises greater than or equal to 98% w/w CBD and less than or equal to other cannabinoids was used in the open label, expanded-access program described in Example 1 below.

In summary the drug substance used in the trials is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 95% CBD w/w, typically greater than 98% w/w.

The *Cannabis sativa* L. plants are grown, harvested, and processed to produce a botanical extract (intermediate) and then purified by crystallization to yield the CBD (botanically derived purified CBD).

The plant starting material is referred to as Botanical Raw Material (BRM); the botanical extract is the intermediate; and the active pharmaceutical ingredient (API) is CBD, the drug substance.

All parts of the process are controlled by specifications. The botanical raw material specification is described in Table B and the CBD API is described in Table C.

**Table B: CBD botanical raw material specification**

| Test | Method | Specification |
|---|---|---|
| Identification:<br>-A | Visual | Complies |

(continued)

| Test | Method | Specification |
|---|---|---|
| -B<br>-C | TLC<br>HPLC/UV | Corresponds to standard (for CBD & CBDA)<br>Positive for CBDA |
| Assay:<br>CBDA + CBD | In-house (HPLC/UV) | NLT 90% of assayed<br>cannabinoids by peak area |
| Loss on Drying | Ph. Eur. | NMT 15% |
| Aflatoxin | UKAS method | NMT 4ppb |
| Microbial:<br>- TVC<br>- Fungi<br>- E.coli | Ph.Eur. | NMT $10^7$cfu/g<br>NMT $10^5$cfu/g<br>NMT $10^2$cfu/g |
| Foreign Matter: | Ph.Eur. | NMT 2% |
| Residual Herbicides and Pesticides | Ph.Eur. | Complies |

Table C: Specification of an exemplary botanically derived purified CBD preparation

| Test | Test Method | Limits |
|---|---|---|
| Appearance | Visual | Off-white / pale yellow crystals |
| Identification A | HPLC-UV | Retention time of major peak corresponds to certified CBD Reference Standard |
| Identification B | GC-FID/MS | Retention time and mass spectrum of major peak corresponds to certified CBD Reference Standard |
| Identification C | FT-IR | Conforms to reference spectrum for certified CBD Reference Standard |
| Identification D | Melting Point | 65 - 67°C |
| Identification E | Specific Optical Rotation | Conforms with certified CBD Reference Standard; -110° to -140° (in 95% ethanol) |
| Total Purity | Calculation | ≥ 98.0% |
| Chromatographic Purity 1 | HPLC-UV | ≥ 98.0% |
| Chromatographic Purity 2 | GC-FID/MS | ≥ 98.0 % |
| CBDA<br>CBDV<br>THC<br>CBD-C4 | HPLC-UV | NMT 0.15% w/w<br>0.2-1.0% w/w<br>0.01-0.1% w/w<br>0.3-0.5% w/w |
| Residual Solvents:<br>Alkane<br>Ethanol | GC | NMT 0.5% w/w<br>NMT 0.5% w/w |
| Residual Water | Karl Fischer | NMT 1.0% w/w |

The purity of the botanically derived purified CBD preparation was greater than or equal to 98%. The botanically derived purified CBD includes THC and other cannabinoids, e.g., CBDA, CBDV, CBD-C1, and CBD-C4.

In some embodiments, the CBD preparation comprises not more than 0.15% THC based on total amount of cannabinoid in the preparation. In some embodiments, the CBD preparation comprises about 0.01% to about 0.1% THC based on total amount of cannabinoid in the preparation. In some embodiments, the CBD preparation comprises about 0.02% to about 0.05% THC based on total amount of cannabinoid in the preparation.

In some embodiments, the CBD preparation comprises about 0.2% to about 1.0% CBDV based on total amount of cannabinoid in the preparation. In some embodiments, the CBD preparation comprises about 0.2% to about 0.8% CBDV based on total amount of cannabinoid in the preparation.

In some embodiments, the CBD preparation comprises about 0.3% to about 0.5% CBD-C4 based on total amount of cannabinoid in the preparation. In some embodiments, the CBD preparation comprises about 0.3% to about 0.4% CBD-C4 based on total amount of cannabinoid in the preparation.

In some embodiments, the CBD preparation comprises about 0.1% to about 0.15% CBD-C1 based on total amount of cannabinoid in the preparation.

Distinct chemotypes of the *Cannabis sativa* L. plant have been produced to maximize the output of the specific chemical constituents, the cannabinoids. Certain chemovars produce predominantly CBD. Only the (-)-trans isomer of CBD is believed to occur naturally. During purification, the stereochemistry of CBD is not affected.

*Production of CBD botanical drug substance*

An overview of the steps to produce a botanical extract, the intermediate, are as follows:

a) Growing
b) Direct drying
c) Decarboxylation
d) Extraction - using liquid $CO_2$
e) Winterization using ethanol
f) Filtration
g) Evaporation

High CBD chemovars were grown, harvested, dried, baled and stored in a dry room until required. The botanical raw material (BRM) was finely chopped using an Apex mill fitted with a 1 mm screen. The milled BRM was stored in a freezer prior to extraction.

Decarboxylation of CBDA to CBD was carried out using heat. BRM was decarboxylated at 115°C for 60 minutes.

Extraction was performed using liquid $CO_2$ to produce botanical drug substance (BDS), which was then crystalized to produce the test material. The crude CBD BDS was winterized to refine the extract under standard conditions (2 volumes of ethanol at -20°C for approximately 50 hours). The precipitated waxes were removed by filtration and the solvent was removed to yield the BDS.

*Production of botanically derived purified CBD preparation*

The manufacturing steps to produce the botanically derived purified CBD preparation from BDS were as follows:

a) Crystallization using $C_5$-$C_{12}$ straight chain or branched alkane
b) Filtration
c) Vacuum drying

The BDS produced using the methodology above was dispersed in $C_5$-$C_{12}$ straight chain or branched alkane. The mixture was manually agitated to break up any lumps and the sealed container then placed in a freezer for approximately 48 hours. The crystals were isolated via vacuum filtration, washed with aliquots of cold $C_5$-$C_{12}$ straight chain or branched alkane, and dried under a vacuum of <10mb at a temperature of 60°C until dry. The botanically derived purified CBD preparation was stored in a freezer at -20°C in a pharmaceutical grade stainless steel container, with FDA food grade approved silicone seal and clamps.

*Physicochemical properties of the botanically derived purified CBD*

The botanically derived purified CBD used in the clinical trial described in the invention comprises greater than or equal to 98% (w/w) CBD and less than or equal to 2% (w/w) of other cannabinoids. The other cannabinoids present are THC at a concentration of less than or equal to 0.1% (w/w); CBD-C1 at a concentration of less than or equal to 0.15% (w/w); CBDV at a concentration of less than or equal to 0.8% (w/w); and CBD-C4 at a concentration of less than or equal to 0.4% (w/w).

The botanically derived purified CBD used additionally comprises a mixture of both trans-THC and cis-THC. It was found that the ratio of the trans-THC to cis-THC is altered and can be controlled by the processing and purification process, ranging from 3.3:1 (trans-THC:cis-THC) in its unrefined decarboxylated state to 0.8:1 (trans-THC:cis-THC) when highly purified.

Furthermore, the cis-THC found in botanically derived purified CBD is present as a mixture of both the (+)-cis-THC and the (-)-cis-THC isoforms.

Clearly a CBD preparation could be produced synthetically by producing a composition with duplicate components.

Example 1 below describes the use of a botanically derived purified CBD in an open label, expanded-access program to investigate the clinical efficacy and safety of purified pharmaceutical cannabidiol formulation (CBD) in the treatment of patients diagnosed with hypoxic ischaemic encephalopathy (HIE) or anoxia at birth.

## EXAMPLE 1: CLINICAL EFFICACY AND SAFETY OF PURIFIED PHARMACEUTICAL CANNABIDIOL (CBD) IN THE TREATMENT OF PATIENTS DIAGNOSED WITH HYPOXIC ISCHAEMIC ENCEPHALOPATHY (HIE) OR ANOXIA AT BIRTH.

*Study design*

Subjects were required to be on one or more AEDs at stable doses for a minimum of two weeks prior to baseline and to have stable vagus nerve stimulation (VNS) settings and ketogenic diet ratios for a minimum of four weeks prior to baseline.

Patients were administered botanically derived purified CBD in a 100 mg/mL sesame oilbased solution most patients were took an initial dose of five milligrams per kilogram per day (mg/kg/day) in two divided doses. Dose was then increased weekly by 5mg/kg/day to a goal of 20 to 25 mg/kg/day.

A maximum dose of 50 mg/kg/day could be utilised for patients who were tolerating the medication but had not achieved seizure control; these patients had further weekly titration by 5mg/kg/day.

There were ten patients in this study, and each received CBD for various durations of time. Modifications were made to concomitant AEDs as per clinical indication.

Seizure frequency, intensity, and duration were recorded by caregivers in a diary during a baseline period of at least 28 days. Changes in seizure frequency relative to baseline were calculated after at least 2 weeks and at defined timepoints of treatment.

*Statistical Methods:*

Patients may be defined as responders if they had more than 50% reduction in seizure frequency compared to baseline. The percent change in seizure frequency was calculated as follows:

$$\frac{\% \text{ change}}{\text{seizure frequency}} = \frac{(\text{weekly seizure frequency } \textit{time interval}) - (\text{weekly seizure frequency } \textit{Baseline})}{(\text{weekly seizure frequency } \textit{Baseline})}$$

The percent change of seizure frequency may be calculated for any time interval where seizure number has been recorded. For the purpose of this example the percent change of seizure frequency for the end of the treatment period was calculated as follows:

$$\frac{\% \text{ reduction}}{\text{seizure frequency}} = \frac{((\text{weekly seizure frequency } \textit{Baseline}) - (\text{weekly seizure frequency } \textit{End})) \times 100}{(\text{weekly seizure frequency } \textit{Baseline})}$$

## Results

*Patient description*

The ten patients enrolled in the open label; expanded-access program were diagnosed with hypoxic ischaemic encephalopathy (HIE) or anoxia at birth. These patients experienced a range of different seizure types including tonic, tonic-clonic, atonic, myoclonic, absence, focal seizures without impairment, focal seizures with impairment, focal seizures with secondary generalisation, and spasms.

The age of patients ranged from 2-14 years, five were male and five were female as detailed in Table 1 below.

**Table 1: Patient demographics, seizure type and concomitant medication**

| Patient Number | Age (years) | Sex | Seizure types | Concomitant AEDs |
|---|---|---|---|---|
| 1 | 2.65 | F | Tonic Spasms | VPA, LTG |
| 2 | 3.09 | F | Absence Spasms | CLB VGB, GBP |
| 3 | 6.02 | M | Tonic Tonic-clonic Focal with impairment | CLB, RFN |
| 4 | 7.66 | M | Tonic Tonic-clonic Atonic | CLB |
| 5 | 8.1 | M | Focal without impairment Focal with secondary generalisation | LEV |
| 6 | 6.57 | M | Tonic-clonic Spasms | CLB, LEV, TPM |
| 7 | 14.0 | F | Tonic-clonic Myoclonic Focal with impairment | CLB, LTG |
| 8 | 8.98 | F | Absence | CLB, VPA, LTG |
| 9 | 7.19 | M | Tonic Myoclonic | CLB, RFN |
| 10 | 9.66 | F | Absence | CLB |
| VPA = valproic acid, LEV = levetiracetam, CLB = clobazam, VGB = vigabatrin, RFN = rufinamide, TPM = topiramate, LTG = lamotrigine, GBP = gabapentin | | | | |

*Study medication and concomitant medications*

Patients on the study were titrated up to various doses of CBD, eight patients were titrated up to at least 25 mg/kg/day (#1-3,5, 7-10), and one patients was titrated up to 50 mg/kg/day (#3).

Patients had tried an average of two AEDs prior to enrolment (range: 1-3 AEDs). Eight patients were taking clobazam (CLB).

*Clinical changes*

Tables 2A-2J illustrate the seizure frequency for each patient as well as the dose of CBD given.

**Table 2A: Seizure frequency data for Patient 1**

| Patient 1 | | | |
|---|---|---|---|
| Time | Seizure Type | | Dose CBD (mg/kg/day) |
| | Tonic | Spasm | |
| Baseline | 1057.0 | 124.0 | - |
| 2 weeks | 1044.0 | 108.0 | 8.3 |
| 4 weeks | 904.0 | 102.8 | 20.8 |
| 8 weeks | 770.0 | 50.8 | 25.2 |
| 16 weeks | 764.8 | 78.0 | 23.4 |
| 24 weeks | 917.2 | 48.0 | 22.9 |
| 36 weeks | 634.0 | 51.2 | 25.7 |
| 48 weeks | 424.8 | 69.2 | 21.8 |

Patient 1 was treated for 48 weeks and experienced a 59.8% reduction in tonic seizures and a 44.2% reduction in spasms over the treatment period.

**Table 2B: Seizure frequency data for Patient 2**

| Patient 2 | | | |
|---|---|---|---|
| **Time** | **Seizure Type** | | **Dose CBD (mg/kg/day)** |
| | **Absence** | **Spasm** | |
| **Baseline** | 64.0 | 580.0 | - |
| **4 weeks** | 52.0 | 440.0 | 20.0 |
| **8 weeks** | 52.0 | 600.0 | 25.0 |
| **16 weeks** | 12.0 | 632.0 | 25.0 |
| **24 weeks** | 24.0 | 580.0 | 25.0 |
| **36 weeks** | 0 | 560.0 | 25.0 |
| **48 weeks** | 0 | 490.0 | 25.0 |
| **60 weeks** | 0 | 196.0 | 25.0 |
| **72 weeks** | 0 | 300.0 | 25.0 |
| **84 weeks** | 0 | 340.0 | 25.0 |

Patient 2 was treated for 84 weeks and experienced a 100% reduction in absence seizures and a 41.4% reduction in spasms over the treatment period.

**Table 2C: Seizure frequency data for Patient 3**

| Patient 3 | | | | |
|---|---|---|---|---|
| **Time** | **Seizure Type** | | | **Dose CBD (mg/kg/day)** |
| | **Tonic** | **Tonic-clonic** | **Focal with impairment** | |
| **Baseline** | 0.0 | 12.0 | 313.0 | - |
| **4 weeks** | 193.0 | 2.0 | 43.0 | 5.0 |
| **8 weeks** | 132.0 | 0 | 132.0 | 20.0 |
| **12 weeks** | 123.0 | 0 | 38.0 | 25.0 |
| **16 weeks** | 120.0 | 6.0 | 46.0 | 25.0 |
| **24 weeks** | 122.0 | 4.8 | 51.2 | 30.0 |
| **36 weeks** | 124.8 | 20.0 | 115.0 | 40.0 |
| **48 weeks** | 136.0 | 11.2 | 38.8 | 50.0 |
| **72 weeks** | 94.7 | 4.0 | 53.0 | 50.0 |
| **84 weeks** | 72.0 | 5.7 | 53.0 | 50.0 |
| **96 weeks** | 70.0 | 11.6 | 46.3 | 50.0 |
| **108 weeks** | 35.0 | 10.4 | 46.4 | 50.0 |
| **120 weeks** | 46.8 | 9.8 | 62.8 | 50.0 |
| **132 weeks** | 54.4 | 12.0 | 77.2 | 50.0 |
| **144 weeks** | 62.4 | 21.2 | 72.0 | 50.0 |

Patient 3 was treated for 144 weeks and experienced a 77% reduction in focal seizures with impairment over the treatment period.

**Table 2D: Seizure frequency data for Patient 4**

| Patient 4 | | | | |
|---|---|---|---|---|
| Time | Seizure Type | | | Dose CBD (mg/kg/day) |
| | Tonic | Tonic-clonic | Atonic | |
| Baseline | 150.0 | 150.0 | 2.0 | - |
| 4 weeks | 4.0 | 13.0 | 0 | 5.0 |
| 8 weeks | 3.0 | 18.0 | 0 | 5.0 |
| 12 weeks | 0 | 10.0 | 0 | 10.0 |
| 24 weeks | 0 | 0.6 | 6.0 | 15.0 |
| 36 weeks | 0 | 0 | 1.7 | 15.0 |
| 48 weeks | 0 | 3.3 | 0 | 15.0 |
| 60 weeks | 0 | 19.3 | 4.0 | 20.0 |

Patient 4 was treated for 60 weeks and experienced a 100% reduction in tonic seizures and an 87.1% reduction in tonic-clonic seizures over the treatment period.

**Table 2E: Seizure frequency data for Patient 5**

| Patient 5 | | | |
|---|---|---|---|
| Time | Seizure Type | | Dose CBD (mg/kg/day) |
| | Focal without impairment | Focal secondary generalisation | |
| Baseline | 1.0 | 104.0 | - |
| 2 weeks | 0 | 114.0 | 10.0 |
| 4 weeks | 2.0 | 106.0 | 20.0 |
| 8 weeks | 1.9 | 112.8 | 25.0 |
| 12 weeks | 2.1 | 111.0 | 25.0 |
| 16 weeks | 3.9 | 125.5 | 25.0 |

Patient 5 was treated for 16 weeks and did not experience a reduction in seizures over the treatment period.

**Table 2F: Seizure frequency data for Patient 6**

| Patient 6 | | | |
|---|---|---|---|
| Time | Seizure Type | | Dose CBD (mg/kg/day) |
| | Tonic-clonic | Spasm | |
| Baseline | 14.0 | 217.0 | - |
| 2 weeks | 10.0 | 100.0 | 10.0 |

Patient 6 was treated for 2 weeks and experienced a 28.6% reduction in tonic-clonic seizures and an 53.9% reduction in spasms over the treatment period.

**Table 2G: Seizure frequency data for Patient 7**

| Patient 7 | | | | |
|---|---|---|---|---|
| Time | Seizure Type | | | Dose CBD (mg/kg/day) |
| | Tonic-clonic | Myoclonic | Focal with impairment | |
| Baseline | 42.0 | 10.0 | 43.2 | - |

(continued)

| Patient 7 | | | |
|---|---|---|---|
| **Time** | **Seizure Type** | | **Dose CBD (mg/kg/day)** |
| | **Tonic-clonic** | **Myoclonic** | **Focal with impairment** | |
| **2 weeks** | 74.0 | 58.0 | 70.0 | 5.0 |
| **4 weeks** | 102.0 | 18.0 | 78.0 | 10.0 |
| **8 weeks** | 86.7 | 65.3 | 65.3 | 20.0 |
| **12 weeks** | 65.0 | 7.0 | 71.0 | 25.0 |

Patient 7 was treated for 12 weeks and experienced a 30% reduction in myoclonic seizures over the treatment period.

### Table 2H: Seizure frequency data for Patient 8

| Patient 8 | | |
|---|---|---|
| **Time** | **Seizure Type Absence** | **Dose CBD (mg/kg/day)** |
| **Baseline** | 7560.0 | - |
| **4 weeks** | 5376.0 | 5.0 |
| **8 weeks** | 4803.0 | 5.0 |
| **12 weeks** | 3514.0 | 10.0 |
| **24 weeks** | 4284.0 | 15.0 |
| **36 weeks** | 2989.3 | 20.0 |
| **48 weeks** | 3360.0 | 25.0 |
| **60 weeks** | 3136.0 | 25.0 |
| **72 weeks** | 2968.0 | 25.0 |
| **84 weeks** | 2718.0 | 25.0 |

Patient 8 was treated for 84 weeks and experienced a 64% reduction in absence seizures over the treatment period.

### Table 2I: Seizure frequency data for Patient 9

| Patient 9 | | | |
|---|---|---|---|
| **Time** | **Seizure Type** | | **Dose CBD (mg/kg/day)** |
| | **Tonic** | **Myoclonic** | |
| **Baseline** | 93.0 | 1.0 | - |
| **2 weeks** | 118.0 | 2.0 | 15.0 |
| **4 weeks** | 88.0 | 1.0 | 20.0 |
| **8 weeks** | 48.0 | 0 | 25.0 |
| **12 weeks** | 92.0 | 0 | 25.0 |
| **16 weeks** | 90.0 | 0 | 25.0 |
| **24 weeks** | 142.4 | 0 | 25.0 |
| **36 weeks** | 53.0 | 0 | 25.0 |
| **48 weeks** | 98.0 | 0 | 25.0 |
| **60 weeks** | 95.6 | 0 | 25.0 |

(continued)

| Patient 9 | | | |
|---|---|---|---|
| **Time** | **Seizure Type** | | **Dose CBD (mg/kg/day)** |
| | **Tonic** | **Myoclonic** | |
| **72 weeks** | 96.0 | 0 | 25.0 |
| **84 weeks** | 99.2 | 0 | 25.0 |
| **108 weeks** | 69.0 | 0 | 25.0 |
| **132 weeks** | 76.8 | 0 | 25.0 |
| **144 weeks** | 79.6 | 0 | 25.0 |

Patient 9 was treated for 144 weeks and experienced a 14.4% reduction in tonic seizures and a 100% reduction in myoclonic seizures over the treatment period.

**Table 2J: Seizure frequency data for Patient 10**

| Patient 10 | | |
|---|---|---|
| **Time** | **Seizure Type** | **Dose CBD (mg/kg/day)** |
| | **Absence** | |
| **Baseline** | 3000.0 | - |
| **4 weeks** | 10.0 | 5.0 |
| **8 weeks** | 1435.0 | 5.0 |
| **12 weeks** | 610.0 | 10.0 |
| **24 weeks** | 304.0 | 15.0 |
| **36 weeks** | 2184.6 | 20.0 |
| **48 weeks** | 1957.3 | 25.0 |
| **60 weeks** | 1520.3 | 25.0 |
| **72 weeks** | 1655.6 | 25.0 |
| **84 weeks** | 1626.6 | 25.0 |
| **96 weeks** | 1678.3 | 25.0 |
| **120 weeks** | 1644.5 | 25.0 |
| **132 weeks** | 1370.8 | 25.0 |

Patient 10 was treated for 132 weeks and experienced a 54.3% reduction in absence seizures over the treatment period.

Overall, patients reported reductions of 14.4-100.0% in seizures over period of treatment with CBD.

CBD was effective in reducing the frequency of the following seizure types: tonic, tonic-clonic, atonic, myoclonic, absence, focal seizures with impairment and spasms. Significantly, two patients became seizure free of tonic seizures after 36 weeks and 12 weeks (patients #2 and 4 respectively) of CBD treatment.

**Conclusions**

These data indicate that CBD was able to significantly reduce the number of seizures associated with hypoxic ischaemic encephalopathy (HIE) or anoxia at birth. Clearly the treatment is of significant benefit in this difficult to treat epilepsy syndrome given the high responder rate experienced in many of the patients.

Of interest is that patients with tonic seizures (patients 2 and 4) who obtained significant benefit whereby both were completely seizure free between 12 and 36 weeks of treatment.

In conclusion, this study signifies the use of CBD for treatment of seizures associated with hypoxic ischaemic encephalopathy (HIE) or anoxia at birth. Seizure types include tonic, tonic-clonic, atonic, myoclonic, absence, focal seizures with impairment and spasms for which seizure frequency rates decreased by significant rates, by 14.4-100.0%.

**References**

1. Lafuente et al. (2016) "Effects of Cannabidiol and Hypothermia on Short-Term Brain Damage in New-Born Piglets after Acute Hypoxia-Ischemia" Frontier in Neuroscience.
2. https://www.abclawcenters.com/blog/2019/07/12/clinical-trial-of-cannabidiol-cbd-in-newborns-with-hypoxic-ischemic-encephalopathy/#:~:text=Research%20has%20shown%20that%20cannabidiol,and%20reduce%20glutamate%2Drelated%20excitotoxicity.&text=Despite%20this%20challenge%2C%20research%20on,approved%20for%20a%20clinical%20trial.
3. Morales et al. (2017) "Use of cannabidiol (RSHO) in the treatment of refractory epilepsy (Lennox-Gastaut Syndrome), experience of 38 cases." Epilepsia; 58, Supplement 5, Special Issue, abstract S53 (p0222)
4. Zollner et al. (2019) "Improving post-hypoxic myclonus using cannabidiol." Seizure, (2019), 67, 38-39
5. Alvarez et al. (2008) "Neuroprotective Effects of the Nonpsychoactive Cannabinoid Cannabidiol in Hypoxic-Ischemic Newborn Piglets." Pediatric Research, 2008, 64(6), 653-658

**Claims**

1. A cannabidiol (CBD) preparation for use in the treatment of absence seizures associated with hypoxic ischaemic encephalopathy (HIE) or anoxia at birth wherein the CBD preparation comprises greater than 95% (w/w) CBD and between 0.01 and 0.15% (w/w) tetrahydrocannabinol (THC).

2. A CBD preparation for use according to claim 1, wherein the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC.

3. A CBD preparation for use according to any of the preceding claims, wherein the CBD preparation is used in combination with one or more concomitant anti-epileptic drugs (AED).

4. A CBD preparation for use according to claim 4, wherein the one or more AED is selected from the group consisting of: valproic acid, levetiracetam, clobazam, vigabatrin, rufinamide, topiramate, lamotrigine and gabapentin.

5. A CBD preparation for use according to any of the preceding claims, wherein the CBD present is isolated from cannabis plant material.

6. A CBD preparation for use according to any of the preceding claims, wherein at least a portion of at least one of the cannabinoids present in the CBD preparation is isolated from cannabis plant material.

7. A CBD preparation for use according to claims 1 to 5, wherein the CBD is present as a synthetic preparation.

8. A CBD preparation for use according to any preceding claim, wherein at least a portion of at least one of the cannabinoids present in the CBD preparation is prepared synthetically.

9. A CBD preparation for use according to any of the preceding claims, wherein the dose of CBD is greater than 5 mg/kg/day.

10. A CBD preparation for use according to any of the preceding claims, wherein the dose of CBD is 20 mg/kg/day.

11. A CBD preparation for use according to any of the preceding claims, wherein the dose of CBD is 25 mg/kg/day.

12. A CBD preparation for use according to any of the preceding claims, wherein the dose of CBD is 50 mg/kg/day.

**Patentansprüche**

1. Cannabidiol (CBD)-Präparat zur Verwendung bei der Behandlung von Absence-Anfällen in Verbindung mit hypoxischer ischämischer Enzephalopathie (HIE) oder Anoxie bei Geburt, wobei das CBD-Präparat mehr als 95 % (w/w) CBD und zwischen 0,01 und 0,15 % (w/w) Tetrahydrocannabinol (THC) umfasst.

2. CBD-Präparat zur Verwendung gemäß Anspruch 1, wobei das CBD-Präparat mehr als oder gleich 98 % (w/w) CBD und weniger als oder gleich 2 % (w/w) andere Cannabinoide umfasst, wobei die weniger als oder gleich 2 % (w/w) anderen Cannabinoide die Cannabinoide Tetrahydrocannabinol (THC); Cannabidiol-C1 (CBD-C1); Cannabidivarin (CBDV); und Cannabidiol-C4 (CBD-C4) umfassen, und wobei das THC als Gemisch von trans-THC und cis-THC vorhanden ist.

3. CBD-Präparat zur Verwendung gemäß einem der vorhergehenden Ansprüche, wobei das CBD-Präparat in Kombination mit einem oder mehreren begleitenden antiepileptischen Arzneimitteln (AED) verwendet wird.

4. CBD-Präparat zur Verwendung gemäß Anspruch 4, wobei das eine oder die mehreren AED ausgewählt sind aus der Gruppe bestehend aus: Valproinsäure, Levetiracetam, Clobazam, Vigabatrin, Rufinamid, Topiramat, Lamotrigin und Gabapentin,

5. CBD-Präparat zur Verwendung gemäß einem der vorhergehenden Ansprüche, wobei das vorliegende CBD aus Cannabispflanzenmaterial isoliert ist.

6. CBD-Präparat zur Verwendung gemäß einem der vorhergehenden Ansprüche, wobei mindestens ein Teil von mindestens einem der in dem CBD-Präparat vorhandenen Cannabinoide aus Cannabispflanzenmaterial isoliert ist.

7. CBD-Präparat zur Verwendung gemäß Anspruch 1 bis 5, wobei das CBD als synthetisches Präparat vorhanden ist.

8. CBD-Präparat zur Verwendung gemäß einem der vorhergehenden Ansprüche, wobei mindestens ein Teil von mindestens einem der in dem CBD-Präparat vorhandenen Cannabinoide synthetisch zubereitet ist.

9. CBD-Präparat zur Verwendung gemäß einem der vorhergehenden Ansprüche, wobei die Dosis von CBD mehr als 5 mg/kg/Tag ist.

10. CBD-Präparat zur Verwendung gemäß einem der vorhergehenden Ansprüche, wobei die Dosis von CBD 20 mg/kg/Tag ist.

11. CBD-Präparat zur Verwendung gemäß einem der vorhergehenden Ansprüche, wobei die Dosis von CBD 25 mg/kg/Tag ist.

12. CBD-Präparat zur Verwendung gemäß einem der vorhergehenden Ansprüche, wobei die Dosis von CBD 50 mg/kg/Tag ist.

**Revendications**

1. Préparation de cannabidiol (CBD) destinée à être utilisée dans le traitement des crises d'absence associées à une encéphalopathie ischémique hypoxique (HIE) ou à une anoxie à la naissance, ladite préparation de CBD comprenant plus de 95 % (p/p) de CBD et entre 0,01 et 0,15 % (p/p) de tétrahydrocannabinol (THC).

2. Préparation de CBD destinée à être utilisée selon la revendication 1, ladite préparation de CBD comprenant au moins 98 % (p/p) de CBD et au plus 2 % (p/p) d'autres cannabinoïdes, dans laquelle les autres cannabinoïdes présents à au plus 2 % (p/p) comprennent les cannabinoïdes : tétrahydrocannabinol (THC) ; cannabidiol-C1 (CBD-C1) ; cannabidivarine (CBDV) ; et cannabidiol-C4 (CBD-C4), et dans laquelle le THC est présent sous forme d'un mélange de trans-THC et de cis-THC.

3. Préparation de CBD destinée à être utilisée selon l'une quelconque des revendications précédentes, ladite préparation de CBD étant utilisée en combinaison avec un ou plusieurs médicaments anti-épileptiques (AED) concomitants.

4. Préparation de CBD destinée à être utilisée selon la revendication 4, dans laquelle le ou les AED sont choisis dans le groupe constitué par : l'acide valproïque, le lévétiracétam, le clobazam, la vigabatrine, le rufinamide, le topiramate, la lamotrigine et la gabapentine.

5. Préparation de CBD destinée à être utilisée selon l'une quelconque des revendications précédentes, dans laquelle le CBD présent est isolé à partir de matière végétale de cannabis.

6. Préparation de CBD destinée à être utilisée selon l'une quelconque des revendications précédentes, dans laquelle au moins une partie d'au moins l'un des cannabinoïdes présents dans la préparation de CBD est isolée à partir de matière végétale de cannabis.

7. Préparation de CBD destinée à être utilisée selon les revendications 1 à 5, dans laquelle le CBD est présent sous forme d'une préparation synthétique,

8. Préparation de CBD destinée à être utilisée selon l'une quelconque des revendications précédentes, dans laquelle au moins une partie d'au moins l'un des cannabinoïdes présents dans la préparation de CBD est préparée de manière synthétique,

9. Préparation de CBD destinée à être utilisée selon l'une quelconque des revendications précédentes, dans laquelle la dose de CBD est supérieure à 5 mg/kg/jour.

10. Préparation de CBD destinée à être utilisée selon l'une quelconque des revendications précédentes, dans laquelle la dose de CBD est de 20 mg/kg/jour.

11. Préparation de CBD destinée à être utilisée selon l'une quelconque des revendications précédentes, dans laquelle la dose de CBD est de 25 mg/kg/jour.

12. Préparation de CBD destinée à être utilisée selon l'une quelconque des revendications précédentes, dans laquelle la dose de CBD est de 50 mg/kg/jour.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2551986 A
- GB 2504263 A
- GB 2574321 A

### Non-patent literature cited in the description

- **SULAK D et al.** *Epilepsy and Behavior*, 2017, vol. 70, 328-333
- **ROGER PERTWEE**. Handbook of Cannabis, 3-15
- **LAFUENTE et al.** Effects of Cannabidiol and Hypothermia on Short-Term Brain Damage in New-Born Piglets after Acute Hypoxia-Ischemia. *Frontier in Neuroscience*, 2016
- **MORALES et al.** Use of cannabidiol (RSHO) in the treatment of refractory epilepsy (Lennox-Gastaut Syndrome), experience of 38 cases. *Epilepsia*, 2017, vol. 58 (5), 0222
- **ZOLLNER et al.** Improving post-hypoxic myclonus using cannabidiol. *Seizure*, 2019, vol. 67, 38-39
- **ALVAREZ et al.** Neuroprotective Effects of the Nonpsychoactive Cannabinoid Cannabidiol in Hypoxic-Ischemic Newborn Piglets. *Pediatric Research*, 2008, vol. 64 (6), 653-658